# EUROPÄISCHE PATENTSCHRIFT

(11) **EP 2 024 378 B1**
(45) Veröffentlichungstag und Bekanntmachung des Hinweises auf die Patenterteilung: **07.09.2011**
(21) Anmeldenummer: 07724809.4
(22) Anmeldetag: 03.05.2007
(51) Int. Cl.: C07H 3/04

(54) **ISOMALTULOSE MIT VERBESSERTER FLIESSFÄHIGKEIT**
ISOMALTULOSE WITH IMPROVED FLOW PERFORMANCE
ISOMALTULOSE AYANT UNE FLUIDITÉ AMÉLIORÉE

(30) Priorität: 08.05.2006 DE 102006022506
(43) Veröffentlichungstag der Anmeldung: 18.02.2009
(73) Patentinhaber: Südzucker Aktiengesellschaft Mannheim/Ochsenfurt, 68165 Mannheim (DE)
(72) Erfinder: KOWALCZYK, Jörg, 67304 Eisenberg/Steinborn (DE); BERNARD, Jörg, 67283 Albsheim (DE); DÖRR, Tillmann, 67591 Hohen-Sülzen (DE)
(74) Vertreter: Schwahn, Hartmut
(86) Internationale Anmeldenummer: PCT/EP2007/003883
(87) Internationale Veröffentlichungsnummer: WO 2007/128483

(56) Entgegenhaltungen:
- EP-A- 1 550 666
- DE-A1- 19 943 491
- DE-A1-102004 052 800
- JP-A- 60 204 709

## Beschreibung

### Beschreibung

Die Erfindung betrifft eine kristalline Isomaltulose, die verbesserte Fließeigenschaften aufweist und nicht zur Zeitverfestigung neigt, Verfahren zur Herstellung dieses Produkts sowie eine neue Verwendung von Isomaltulose.

### Stand der Technik

Isomaltulose (auch als Palatinose® bezeichnet) ist ein Disaccharid mit α-1,6-glycosidischer Verknüpfung von Glucose und Fructose (6-O-α-D-Glucopyranosyl-Fructofuranose). Isomaltulose wird in der Lebensmittelindustrie und in der pharmazeutischen Industrie als Süßungsmittel, Trägerstoff und/oder Füllstoff eingesetzt. Ein wichtiges Einsatzgebiet von Isomaltulose ist der Ersatz von Saccharose. Isomaltulose wird wie Saccharose oder andere Zuckeraustauschstoffe oft in kristalliner Form als loses Schüttgut gelagert und verarbeitet. Kristalline Isomaltulose sollte möglichst gute Lagerfähigkeit und Fließeigenschaften wie andere Zuckeraustauschstoffe aufweisen, um in für solche Stoffe geeignete und angepasste Verarbeitungsanlagen eingespeist werden zu können, ohne dass größere verfahrenstechnische Anpassungen oder bauliche Veränderungen an den Anlagen erforderlich werden.

Im Gegensatz zu anderen kristallinen Zuckeraustauschstoffen ist Isomaltulose wenig hygroskopisch; demgemäß ist eine hohe Lagerstabilität zu erwarten. Unerwarteterweise zeigt Isomaltulose jedoch den nachteiligen Effekt der sogenannten "Zeitverfestigung". Bei diesem Phänomen "verbacken" die Isomaltulosekristalle miteinander zu Klumpen oder Agglomeraten. Solche Klumpen oder Agglomerate behindern die Fließfähigkeit des Produkts bei der Weiterverarbeitung. Beispielsweise wird Isomaltulose in Säcken oder sogenannten "big packs" gelagert und der Inhalt der Säcke zur maschinellen Verarbeitung über Einfülltrichter oder Schütten den Anlagen zugeführt. Die gebildeten Agglomerate erschweren das Entleeren der Säcke und behindern das Einfließen des Schüttguts in die Einfülltrichter. In der Regel werden daher zusätzliche mechanische Bearbeitungsschritte notwendig, um die gelagerte und agglomerierte Isomaltulose der maschinellen Verarbeitung zuzuführen.

Alternativ wird Isomaltulose in Schüttgutsilos gelagert. Dort tritt ebenfalls Zeitverfestigung auf. So neigt Isomaltulose in Kernflusssilos zu Schachtbildung. Eine Silolagerung von Isomaltulose ist deutlich erschwert und in bestimmten Silotypen unzweckmäßig. Zusätzliche Vorkehrungen bei der Silolagerung wie periodische Umwälzungen innerhalb des Silos müssen getroffen werden.

Systematische Untersuchungen zeigen, dass massive Zeitverfestigungen bereits nach kurzen Lagerzeiten von etwa 48 Stunden auftreten. Weiter zeigt sich, dass die weitere Verlängerung der Lagerdauer über mehrere Tage hinweg (beispielsweise 240 Stunden) den initialen Umfang der Zeitverfestigung nur noch geringfügig vergrößert. Im Vergleich zu anderen Schüttgütern, die bekanntermaßen ebenfalls zur Zeitverfestigung neigen, tritt die Zeitverfestigung bei Isomaltulose nachteiligerweise sehr schnell auf. Die Zeitverfestigung von Isomaltulose ist außerdem im Wesentlichen unabhängig vom üblichen Feuchtigkeitsgehalt gelagerter Isomaltulose und von üblichen Lagerbedingungen. Feuchtegehalt und Lagerbedingungen haben nachteiligerweise keinen ausreichenden Einfluss auf Zeitverfestigung und Fließfähigkeit der Isomaltulose. Es sind keine üblicherweise verfügbaren Lagerbedingungen bekannt, bei denen die unerwünsche Zeitverfestigung ausreichend reduziert wird.

Es besteht der Bedarf, kristalline Isomaltulose als Schüttgut in fließfähiger und silofähiger Form zu erhalten, ohne dass bei der Lagerung eine nachteilige Zeitverfestigung auftritt.

Um die Fließfähigkeit von Schüttgütern für die Lagerung und Verarbeitung im Lebensmittelbereich und in der Pharmazeutik zu erhalten, werden in der Regel sogenannte "Antibackmittel" oder "Rieselhilfsmittel" (anticaking agent) beigefügt. Bekannte Rieselhilfsmittel sind Silikate, Aluminosilikate, Polysyloxane, Phosphate, Natriumhydrogencarbonat oder Stärkepulver. Nachteilig bei diesen Hilfsstoffen ist, dass sie das Ausgangsprodukt verunreinigen und/oder chemische Eigenschaften aufweisen, bei der Verarbeitung des Produkts zu Nachteilen führt. Außerdem ist es erforderlich, solche Hilfsstoffe im fertigen Lebensmittelprodukt oder pharmazeutischen Produkt zu deklarieren.

### Aufgabenstellung

Ausgehend vom Stand der Technik besteht das der vorliegenden Erfindung zugrundeliegende technische Problem im Wesentlichen darin, fließfähige kristalline Isomaltulose bereitzustellen, die ohne den Zusatz von Fremd- und Hilfsstoffen wie Antibackmittel und Rieselhilfsmittel bei der Lagerung ihre Fließfähigkeit behält und keine Tendenz zur Zeitverfestigung besitzt.

Weiter besteht das technische Problem, ein verbessertes Antibackoder Rieselhilfsmittel bereitzustellen, das die Fließfähigkeit von kristalliner Isomaltulose verbessert und die Neigung zur Zeitverfestigung vermindert oder unterdrückt.

Das zugrundeliegende technische Problem wird erfindungsgemäß gelöst durch die Bereitstellung einer kristallinen Isomaltulose, die einen Anteil von mindestens 1 bis maximal 20 Gew.-% (bezogen auf den Gesamttrockensubstanzgehalt des Produkts) feinpulvrige Isomaltulose, das heißt Isomaltulose-Feinanteil mit einer Korngröße von weniger als 100 µm, insbesondere weniger als 50 µm, enthält.

Die Erfindung besteht also darin, in bekannter kristalliner Isomaltulose, wie sie bevorzugt in an sich bekannter Weise durch Isomerisierung aus Saccharose und Kristallisation erhalten werden kann, einen bestimmten Anteil an feinpulvriger Isomaltulose vorzusehen. Unter "kristalliner Isomaltulose" wird pulverförmiges granuläres Schüttgut aus Isomaltulose verstanden. Herkömmliche kristalline Isomaltulose weist Isomaltulose-Kristalle mit einer Korngröße von etwa 0,2 bis 0,6 mm auf. Unter "Feinpulvriger Isomaltulose" beziehungsweise "Isomaltulose-Feinanteil" wird vorliegend kristalline Isomaltulose in Pulver- oder Puderform verstanden, welche eine Korngröße von weniger als 100 µm, insbesondere weniger als 50 µm aufweist. Insbesondere bedeutet dies, dass die feinpulvrige Isomaltulose eine bestimmte Korngrößenverteilung aufweist. Bei einer mittels Laserbeugungsmethode (Gerät: Malvern Mastersizer 2000^{®}) bei trockener Dispergierung in Luft mit 0,6 bar Dispergierdruck gemessenen Korngrößenverteilung weist eine erfindungsgemäß eingesetzte feinpulvrige Isomaltulose vorzugsweise folgende Rückstandssummen auf: 5 % (d05) = etwa 44 µm, 50 % (d50) = etwa 15 µm, 95 % (d95) = etwa 1 µm (Prozentangaben beziehen sich auf Volumen-Prozent). Dies bedeutet, dass nur 5 Vol.-% des gesamten Partikelvolumens größer sind als etwa 44 µm. Bevorzugt weist die feinpulvrige Isomaltulose daher zu wesentlichen Teilen, das heißt zu mehr als 95 Vol.-%, eine Korngröße von unter etwa 44 µm auf; vor allem besitzt die feinpulvrige Isomaltulose eine Korngröße von stets weniger als 100 µm, insbesondere weniger als 50 µm. Korngrößen von 100 µm oder mehr treten dabei in der Regel nicht auf; ihr Anteil ist vernachlässigbar gering, besonders aber weniger als 1 Vol.-% und bevorzugt weniger als 0,5 Vol.-%.

Überraschenderweise bewirkt der erfindungsgemäß vorgesehene Isomaltulose-Feinanteil im Produkt, dass die kristalline Isomaltulose bei der Lagerung keine nachteilige Zeitverfestigung mehr zeigt und ihre Fließfähigkeit dauerhaft behält. Überraschenderweise ist der vorteilhafte Effekt bereits bei einem Isomaltulose-Feinanteil von etwa 1 Gew.-% am Gesamtprodukt stark ausgeprägt. Bei einem Feinanteil von etwa 5 Gew.-% ist der vorteilhafte Effekt maximal. Bei einem Feinanteil von mehr als etwa 20 Gew.-% tritt der vorteilhafte Effekt soweit in den Hintergrund, dass das zugrundeliegende technische Problem nicht mehr ausreichend gelöst werden kann. Bevorzugt ist ein Isomaltulose-Feinanteil von 1 bis 10 Gew.-%; besonders bevorzugt ist ein Isomaltulose-Feinanteil von 1 bis 5 Gew.-% (bezogen auf Gesamttrockensubstanz). Besonders bevorzugt ist ein Isomaltulose-Feinanteil von 1 bis 4 Gew.-%. Besonders bevorzugt ist ein Isomaltulose-Feinanteil von 1 bis 3 Gew.-%. Besonders bevorzugt ist ein Isomaltulose-Feinanteil von 1 bis 2 Gew.-%. Besonders bevorzugt ist ein Isomaltulose-Feinanteil von etwa 1 Gew.-%.

In einer bevorzugten Ausführungsform weist die erfindungsgemäße kristalline Isomaltulose ohne den erfindungsgemäßen Feinanteil eine Kristallgröße von etwa 0,2 bis etwa 0,6 mm, bevorzugt von etwa 0,3 bis etwa 0,45 mm, auf. Dies entspricht der Kristallgrößenverteilung von aus Isomerisierung von Saccharose und Kristallisation in an sich bekannter Weise erhaltener kristalliner Isomaltulose.

Eine bevorzugte Ausführung der Erfindung ist eine kristalline Isomaltulose, die einen Isomaltulose-Feinanteil mit einer Korngröße von weniger als 100 µm, insbesondere weniger als 50 µm, von 1 bis 5 Gew.-% (bezogen auf Gesamttrockensubstanz) und einen Anteil an kristalliner Isomaltulose mit einer Kristallgröße von etwa 0,3 bis etwa 0,45 mm von 99 bis 95 Gew.-% (bezogen auf Gesamttrockensubstanz) enthält oder bevorzugt daraus besteht.

Die erfindungsgemäße Isomaltulose ist reine Isomaltulose. Es versteht sich, dass dem erfindungsgemäßen Isomaltulose-Produkt zur weiteren Verbesserung der Verarbeitungsfähigkeit zusätzlich weitere bekannte Hilfsmittel wie Antibackmittel oder Rieselhilfsmittel beigefügt werden können.

Ein weiterer Gegenstand der Erfindung ist demgemäß auch ein Verfahren zur Herstellung reiner, fließfähiger kristalliner Isomaltulose, die insbesondere nicht zur Zeitverfestigung neigt. Das erfindungsgemäße Verfahren ist dadurch gekennzeichnet, dass, bevorzugt auf herkömmliche Weise gewinnbare, kristalline Isomaltulose mit einer Kristallgröße von etwa 0,2 bis etwa 0,6 mm, bevorzugt von etwa 0,3 bis etwa 0,45 mm, mit einem erfindungsgemäßen Anteil von 1 bis 20 Gew.-%, bevorzugt von 1 bis 10 Gew.-%, besonders bevorzugt von 1 bis 5 Gew.-%, weiter bevorzugt von 1 bis 4 Gew.-%, 1 bis 3 Gew.-%, 1 bis 2 Gew.-%, 1 Gew.-% oder, (bezogen auf Gesamttrockensubstanz) feinpulvriger Isomaltulose in Verbindung gebracht, das heißt vermischt, wird und so eine fließfähige, lagerbeständige kristalline Isomaltulose erhalten wird. Vorzugsweise wird unter Rühren intensiv vermischt. Das Vermischen findet während der Produktion des kristallinen Isomaltuloseanteils statt oder unmittelbar vor der Abfüllung oder Lagerung. In einer weiteren bevorzugten Ausführung werden die Prozessparameter so gewählt oder solche weiteren Maßnahmen vorgesehen, dass der erfindungsgemäß vorgesehene Feinanteil in der kristallinen Isomaltulose bei der Herstellung entsteht oder bereits entstandener im Produkt verbleibt.

In jedem Fall wird ein reines Isomaltulose-Produkt erhalten, das, von üblichen Verunreinigungen abgesehen, allein aus Isomaltulose besteht.

Möglich ist auch ein fließfähiges und lagerstabiles Isomaltulosehaltiges Gemisch, das den erfindungsgemäßen Feinanteil an Isomaltulose aufweist. Es handelt sich dabei besonders um eine Kombination von Isomaltulose mit mindestens einem weiteren Kohlenhydrat, besonders Fruktose, Glukose, Saccharose, Trehalulose, Isomaltose, Isomelizitose, Oligosacchariden mit einem Polymerisationsgrad von 3 oder 4, bevorzugt Inulin und/oder Oligofructose, oder Kohlenhydratalkohol, besonders Mannit, Sorbit, Xylit, Isomalt, oder Gemischen davon. In einer bevorzugten Variante enthält das Gemisch Isomaltulose und Fruktose oder besteht daraus, in einer weiteren bevorzugten Variante enthält das Gemisch Isomaltulose und Glukose oder besteht daraus, in einer weiteren bevorzugten Variante enthält das Gemisch Isomaltulose und Saccharose oder besteht daraus, in einer weiteren bevorzugten Variante enthält das Gemisch Isomaltulose und Trehalulose oder besteht daraus, in einer weiteren Variante enthält das Gemisch Isomaltulose und Isomaltose oder besteht daraus, in einer bevorzugten weiteren bevorzugten Variante enthält das Gemisch Isomaltulose und Isomelizitose oder besteht daraus, in einer weiteren bevorzugten Variante enthält das Gemisch Isomaltulose und Oligosaccharide mit einem Polymerisationsgrad von 3 oder 4 oder mehr oder besteht daraus.

Weiter wurde gefunden, dass feinpulverige Isomaltulose auch bei anderen ähnlichen, insbesondere kristallinen, Schüttgütern zur Verbesserung der Fließfähigkeit und zur Verhinderung der Zeitverfestigung eingesetzt werden kann. So zeigt sich, dass der erfindungsgemäße Isomaltulose-Feinanteil die Verarbeitungsfähigkeit oder Fließfähigkeit von kristallinen Produkten wie Saccharose, Glucose, Fructose, Isomalt sowie anderen festen, pulverförmigen Mono,- Di-, Oligo-, Polysacchariden und -saccharidalkoholen verbessert und gegebenenfalls deren Neigung zur Zeitverfestigung unterdrückt oder vermindert.

Ein weiterer Gegenstand der Erfindung ist daher auch die Verwendung der feinpulvrigen Isomaltulose als Rieselhilfsmittel in kristallinen Schüttgûten ausgewâhlt aus der Gruppe bestehend aus pulverförmigen Mono-, Di, Oligo-, Polysaccharides und - saccharidalkoholen Ein weiterer Gegenstand der Erfindung ist die Verwendung der feinpulvrigen Isomaltulose als Antibackmittel in kristallinen Schüttgûten ausgewâhlt aus der Gruppe bestehend aus pulver formigen Mono-, Di, Oligo-, Polysaccharides und - saccharidalkoholen. Erfindungsgemäß wird der Isomaltulose-Feinanteil verwendet, um die Fließfähigkeit der kristallinen Schüttgûten ausgewâhlt aus der fruppe bestehend aus pulver formigen Mons-, Di, Oligo-, Polysaccharides und -saccharidallcholen vor allem für die maschinelle Verarbeitung zu verbessern und/oder diese, insbesondere bei der Lagerung, zu erhalten. Bevorzugt wird den kristallinen Schüttgûten ausgewâhlt aus der Gruppe bestehend aus pulver formigen Mono-, Di, Oligo-, Polysaccharides und -saccharidalkoholen die feinpulvrige Isomaltulose als Rieselhilfsmittel oder Antibackmittel in einem Anteil von 1 bis 20 Gew.-%, bevorzugt 1 bis 10 Gew.-%, besonders bevorzugt 1 bis 5 Gew.-%, weiter bevorzugt von 1 bis 4 Gew.-%, 1 bis 3 Gew.-%, 1 bis 2 Gew.-% oder etwa 1 Gew.-%, (bezogen auf Gesamttrockensubstanz) zugesetzt.

Die Erfindung wird anhand des nachfolgenden Beispiels näher erläutert, ohne dass dieses beschränkend zu verstehen ist.

### Ausführungsbeispiel

### Lagertest von Isomaltulose

Es wurden Isomaltulose-Chargen aus der laufenden Produktion für die Verwendung in der Lebensmittelindustrie untersucht. Dazu wurden die Proben aliquotiert und alle zunächst für 12 Stunden im Trockenschrank bei 60°C und unter Vakuum gelagert. Sofort anschließend wurden der Wassergehalt und die Wasseraktivität (a_{w}-Wert) der Proben ermittelt. Diese Werte dienten als Referenzwert (Nullprobe).

Es wurden folgende Lagerbedingungen gewählt:
Lagertemperatur: 30 °C
45 % relative Feuchte oder 60 % relative Feuchte
Lagerdauer: 3 Wochen
Lagerung ohne mechanische Belastung (Belastung allein durch Eigengewicht der Probe);
Lagerung unter zusätzlicher mechanischer Belastung:
10 kg Last auf eine kreisrunde Fläche von 10 cm Druchmesser;
etwa 125 MPa

### a) Vergleichsexperiment

Die Ergebnisse des Lagertests sind in Tabelle 1 dargestellt. Die Ergebnisse zeigen, dass nach 3 Wochen Lagerdauer kein signifikanter Unterschied gegenüber der Nullprobe in Hinblick auf Wassergehalt und Wasseraktivität festzustellen war. Isomaltulose ist nicht hygroskopisch. Unerwarteterweise traten jedoch Verklumpungen und Verhärtungen der kristallinen Isomaltulose auf.

**Tabelle 1:**

| **Lagerbedingung** | **Wassergehalt** | **Wasseraktivität** | **Zustand der Probe** |
|---|---|---|---|
| **[rel. Feuchte]** | **[%]** | **[a_{w}-Wert]** | |
| Nullprobe | 5.32 | 0,41 | Probe ist voll fließfähig; keine Klumpen |
| 45; ohne Last | 5,33 | 0,43 | bereits nach wenigen Tagen tritt Klumpenbildung auf |
| 45; mit Last | 5,39 | 0,41 | bereits nach wenigen Tagen tritt Klumpenbildung auf; der Effekt ist unter Last stärker als ohne Last |
| 60; ohne Last | 5,44 | 0,44 | Klumpenbildung |
| 60; mit Last, | 5,35 | 0,48 | starke Klumpenbildung; der Effekt ist unter Last stärker als ohne Last |

### b) Isomaltulose mit 5 Gew.-% Feinanteil (erfindungsgemäß)

In einem weiteren Versuchsansatz wurde die aus der laufenden Produktion entnommene Isomaltulose mit 5 Gew.-% (bezogen auf Gesamttrockensubstanzgehalt) feinpulvrige Isomaltulose mit Korngrößen von weniger als 100 µm versetzt und gut durchmischt.

Die feinpulvrige Isomaltulose wies folgende Korngrößen auf:

| | |
|---|---|
| 5 Vol.-% (d05): | 44 µm |
| 50 Vol.-% (d50) : | 15 µm |
| 95 Vol.-% (d95) : | 1 µm |

(Laserbeugungsmethode; Malvern Mastersizer 2000®; Trockendispergierung in Luft; 0,6 bar Dispergierdruck)

Anschließend wurden die Proben aliquotiert, Nullproben bestimmt und wie vorstehend (siehe a)) beschrieben für 3 Wochen gelagert. Die Ergebnisse sind in Tabelle 2 dargestellt. Über die gesamte Lagerdauer von 3 Wochen im Schüttgut waren keine nennenswerten Verklumpungen oder Verhärtungen festzustellen. Auch unter Last zeigten die Proben noch gute Flieseigenschaften. Eventuell gebildete kleinere Klümpchen zerfielen ohne Kraftaufwand.

**Tabelle 2:**

| **Lagerbedingung** | **Wassergehalt** | **Wasseraktivität** | **Zustand der Probe** |
|---|---|---|---|
| **[rel. Feuchte]** | **[%]** | **[a_{w}-Wert]** | |
| Nullprobe | 5,25 | 0,27 | Probe ist voll fließfähig; keine Klumpen |
| 45; ohne Last | 5,15 | 0,39 | Probe ist gut fließfähig; es werden keine Klumpen gebildet |
| 45; mit Last | - | - | Proben sind gut fließfähig; kleinere vorhandene Klümpchen zerfallen sofort |
| 60; ohne Last | 5,15 | 0,45 | Proben sind gut fließfähig; keine Klumpen vorhanden |
| 60; mit Last | - | - | Proben sind gut fließfähig; kleine Klümpchen zerfallen sofort |

### c) Isomaltulose mit 20 Gew.% Feinanteil (erfindungsgmäß)

In einem weiteren Versuchsansatz wurde die aus der laufenden Produktion entnommene Isomaltulose mit 20 Gew.-% (bezogen auf Gesamttrockensubstanzgehalt) Feinpudriger Isomaltulose mit Korngrößen von weniger als 100 µm (siehe b)) versetzt und gut durchmischt. Anschließend wurden die Proben aliquotiert, Nullproben bestimmt und wie vorstehend beschrieben für 3 Wochen gelagert.

Die Ergebnisse sind in Tabelle 3 dargestellt. Auch bei Zusatz von 20 Gew.-% feinpulvriger Isomaltulose waren noch gute Fließeigenschaften festzustellen. Kleinere gebildete Klümpchen zerfielen ohne Kraftaufwand. Die kristalline Isomaltulose ist in dieser Form gut Rieselfähig. Die Rieselfähigkeit ist jedoch geringer wie unter Zusatz von nur 5 Gew.-% feinpulvriger Isomaltulose (siehe b)).

**Tabelle 3:**

| **Lagerbedingung** | **Wassergehalt** | **Wasseraktivität** | **Zustand der Probe** |
|---|---|---|---|
| **[rel. Feuchte]** | **[%]** | **[a_{w}-Wert]** | |
| Nullprobe | 5,16 | 0,19 | Probe ist voll fließfähig; keine Klumpen |
| 45; ohne Last | 5,19 | 0,39 | Probe ist gut fließfähig; es werden keine Klumpen gebildet |
| 45; mit Last | - | - | Proben sind gut fließfähig; kleinere vorhandene Klümpchen zerfallen sofort |
| 60; ohne Last | 5,19 | 0,46 | Proben sind gut fließfähig; keine Klumpen vorhanden; feuchter Eindruck |
| 60; mit Last | - | - | Proben sind gut fließfähig; kleine Klümpchen zerfallen sofort; feuchter Eindruck |

## Patentansprüche

1. Kristalline Isomaltulose, enthaltend 1 bis 20 Gew.-% (bezogen auf Gesamttrockensubstanz) Isomaltulose-Feinanteil mit einer Korngröße von weniger als 100 µm.

2. Kristalline Isomaltulose nach Anspruch 1, enthaltend 1 bis 10 Gew.-% (bezogen auf Gesamttrockensubstanz) Isomaltulose-Feinanteil.

3. Kristalline Isomaltulose nach Anspruch 1 oder 2,
enthaltend 1 bis 5 Gew.-% (bezogen auf Gesamttrockensubstanz) Isomaltulose-Feinanteil.

4. Kristalline Isomaltulose nach einem der vorstehenden Ansprüchen, wobei der nicht dem Feinanteil zuzurechnende kristalline Anteil eine Kristallgröße von 0,2 bis 0,6 mm aufweist.

5. Kristalline Isomaltulose nach Anspruch 4, wobei der nicht dem Feinanteil zuzurechnende kristalline Anteil eine Kristallgröße von 0,3 bis 0,45 mm aufweist.

6. Isomaltulose, bestehend aus:
1 bis 5 Gew.-% (bezogen auf Gesamttrockensubstanz) Feinanteil mit einer Korngröße von weniger als 100 µm und
99 bis 95 Gew.-% (bezogen auf Gesamttrockensubstanz) kristalliner Anteil mit einer Kristallgröße von 0,3 bis 0,45 mm.

7. Verfahren zur Herstellung fließfähiger kristalliner Isomaltulose, wobei kristalline Isomaltulose mit einer Kristallgröße von 0,2 bis 0,6 mm mit in einem Anteil von 1 bis 20 Gew.-% (bezogen auf Gesamttrockensubstanz) an feinpulvriger Isomaltulose mit einer Korngröße von weniger als 100 µm vermischt wird.

8. Verwendung von feinpulvriger Isomaltulose mit einer Korngröße von weniger als 100 µm zur Verbesserung und/oder Erhalt der Fließfähigkeit von kristallinen Schüttgütern ausgewählt aus der Gruppe bestehend aus pulverförmigen Mono-, Di-, Oligo-, Polysacchariden und -saccharidalkoholen.

9. Verwendung nach Anspruch 8, wobei die feinpulvrige Isomaltulose den kristallinen Schüttgütern in einem Anteil von 1 bis 20 Gew.-% (bezogen auf Gesamttrockensubstanz) zugesetzt wird.

## Claims

1. Crystalline isomaltulose containing 1 to 20 wt% (referred to total dry substance) isomaltulose fine fraction with a particle size of less than 100 µm.

2. Crystalline isomaltulose according to claim 1, containing 1 to 10 wt% (referred to total dry substance) isomaltulose fine fraction.

3. Crystalline isomaltulose according to claim 1 or 2, containing 1 to 5 wt% (referred to total dry substance) isomaltulose fine fraction.

4. Crystalline isomaltulose according to one of the preceding claims, wherein the crystalline fraction not belonging to the fine fraction has a crystal size from 0.2 to 0.6 mm.

5. Crystalline isomaltulose according to claim 4, wherein the crystalline fraction not belonging to the fine fraction has a crystal size from 0.3 to 0.45 mm.

6. Isomaltulose containing:
1 to 5 wt% (referred to total dry substance) fine fraction with a particle size of less than 100 µm and
99 to 95 wt% (referred to total dry substance) crystalline fraction with a particle size from 0.3 to 0.45 mm.

7. Method for production of flowable crystalline isomaltulose, wherein crystalline isomaltulose with a particle size from 0.2 to 0.6 mm is mixed with a fraction of 1 to 20 wt% (referred to total dry substance) of fine-powdered isomaltulose with a particle size of less than 100 µm.

8. Use of fine-powdered isomaltulose with a particle size of less than 100 µm to improve and/or obtain flowability of crystalline bulk products selected from the group consisting of powdered mono-, di-, oligo-, polysaccharides and -saccharide alcohols.

9. Use according to claim 8, wherein the fine-powdered isomaltulose is added to the crystalline bulk products in an amount of 1 to 20 wt% (referred to total dry substance).

## Revendications

1. Isomaltulose cristallin contenant 1 à 20 % en poids (par rapport à la matière sèche totale) de particules fines d'isomaltulose présentant une taille de particule inférieure à 100 µm.

2. Isomaltulose cristallin selon la revendication 1, contenant 1 à 10 % en poids (par rapport à la matière sèche totale) de particules fines d'isomaltulose.

3. Isomaltulose cristallin selon la revendication 1 ou 2, contenant 1 à 5 % en poids (par rapport à la matière sèche totale) de particules fines d'isomaltulose.

4. Isomaltulose cristallin selon l'une des revendications précédentes, dans lequel les particules cristallines non comptées parmi les particules fines présentent une taille de cristaux de 0,2 à 0,6 mm.

5. Isomaltulose cristallin selon la revendication 4, dans lequel les particules cristallines non comptées parmi les particules fines présentent une taille de cristaux de 0,3 à 0,45 mm.

6. Isomaltulose cristallin composé de :
1 à 5 % en poids (par rapport à la matière sèche totale) de particules fines présentant une taille de particule inférieure à 100 µm et
99 à 95 % en poids (par rapport à la matière sèche totale) de particules cristallines présentant une taille de cristaux de 0,3 à 0,45 mm.

7. Procédé de fabrication d'isomaltulose cristallin fluide, dans lequel de l'isomaltulose cristallin présentant une taille de cristaux de 0,2 à 0,6 mm est mélangé avec une fraction de 1 à 20 % en poids (par rapport à la matière sèche totale) d'isomaltulose finement poudreux présentant une taille de particule inférieure à 100 µm.

8. Utilisation d'isomaltulose finement poudreux présentant une taille de particule inférieure à 100 µm pour améliorer et/ou obtenir la fluidité de produits cristallins en vrac sélectionnés dans le groupe consistant en mono-, di-, oligo-, polysaccharides et alcools de mono-, di-, oligo-, polysaccharides poudreux.

9. Utilisation selon la revendication 8, dans laquelle l'isomaltulose finement poudreux est ajouté aux produits cristallins en vrac dans une proportion de 1 à 20 % en poids (par rapport à la matière sèche totale).
